(19) Europäisches Patentamt / European Patent Office / Office européen des brevets

(11) **EP 4 112 085 B1**

(12) **EUROPEAN PATENT SPECIFICATION**

(45) Date of publication and mention
of the grant of the patent:
**11.12.2024   Bulletin 2024/50**

(21) Application number: **21859650.0**

(22) Date of filing: **23.04.2021**

(51) International Patent Classification (IPC):
***A61K 51/12*** *(2006.01)*   ***A61K 51/04*** *(2006.01)*
***A61P 35/00*** *(2006.01)*

(52) Cooperative Patent Classification (CPC):
**A61K 51/1217; A61K 51/1251;** A61K 2123/00

(86) International application number:
**PCT/CN2021/089200**

(87) International publication number:
**WO 2022/041803 (03.03.2022 Gazette 2022/09)**

(54) **VISUALIZED RADIOACTIVE CARBON MICROSPHERE, AND PREPARATION METHOD AND APPLICATION THEREOF**

VISUALISIERTE RADIOAKTIVE KOHLENSTOFFMIKROKUGEL SOWIE HERSTELLUNGSVERFAHREN UND ANWENDUNG DAVON

MICROSPHÈRE DE CARBONE RADIOACTIVE VISUALISÉE, ET PROCÉDÉ DE PRÉPARATION ET APPLICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2020   CN 202010867997**

(43) Date of publication of application:
**04.01.2023   Bulletin 2023/01**

(73) Proprietor: **Chengdu New Radiomedicine Technology Co., Ltd.**
**Chengdu, Sichuan 610200 (CN)**

(72) Inventors:
- **ZHAO, Xiaosheng**
  **Ganzhou, Jiangxi 342400 (CN)**
- **ZHANG, Jun**
  **Guangyuan, Sichuan 628300 (CN)**
- **ZHANG, Chixiang**
  **Chengdu, Sichuan 610036 (CN)**
- **LU, Jing**
  **Chongqing 408500 (CN)**
- **LIU, Qiangqiang**
  **Chengdu, Sichuan 610000 (CN)**
- **LUO, Xianjin**
  **Yiliang County Zhaotong**
  **Yunnan 657000 (CN)**

- **MA, Wenliang**
  **Chengdu, Sichuan 610000 (CN)**
- **GE, Qiang**
  **Chengdu, Sichuan 610041 (CN)**
- **CAI, Jiming**
  **Chengdu, Sichuan 610041 (CN)**

(74) Representative: **Ipside**
**7-9 Allée Haussmann**
**33300 Bordeaux Cedex (FR)**

(56) References cited:
WO-A1-2009/129577    CN-A- 107 303 286
CN-A- 107 715 122    CN-A- 107 715 123
CN-A- 107 715 124    CN-A- 111 920 968

- G FERRO-FLORES ET AL: "Multifunctional Radiolabeled Nanoparticles for Targeted Therapy", CURRENT MEDICINAL CHEMISTRY, vol. 21, no. 1, 1 January 2014 (2014-01-01), pages 124 - 138, XP055207519, DOI: 10.2174/09298673113209990218
- FISCHER GABRIEL ET AL.: "89Zr, a Radiometal Nuclide with High Potential for Molecular Imaging with PET: Chemistry, Applications and Remaining Challenges", MOLECULES, vol. 18, 3 June 2013 (2013-06-03), pages 6469 - 6490, XP055147850, DOI: 10.3390/molecules18066469

## Description

## Technical Field

The present invention relates to the technical field of medicines, and in particular to a visualized radioactive carbon microsphere suspension, and a preparation method and use thereof.

## Background Art

In recent years, local, minimally invasive radioactive treatment has played an increasingly important role in clinical treatment, and has been more and more widely used especially in the treatment of unresectable liver cancer, prostate cancer, kidney cancer and other solid tumors.

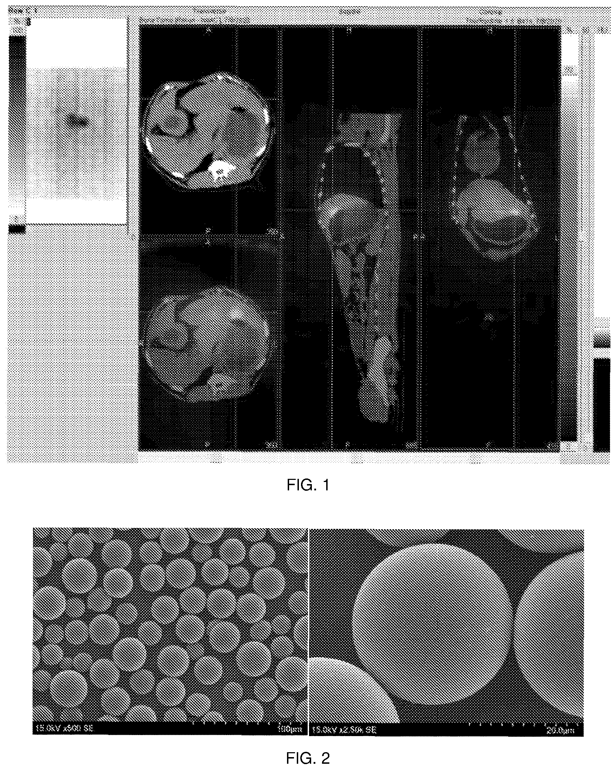

Currently, the radioactive microsphere products for local, minimally invasive radioactive treatment of advanced liver cancer in clinic mainly include yttrium [$^{90}$Y] resin microspheres SIR-Spheres® (Sirtex Medical Limited, Australia) and yttrium [$^{90}$Y] glass microspheres TheraSphere®(BTG, UK). Because the radioactive yttrium [$^{90}$Y] nuclide is a pure beta nuclide, it can only generate gamma rays for imaging through bremsstrahlung, so only blurred images (as shown in Fig. 1) are available in single photon emission computed tomography (SPECT) and positron emission tomography (PET), and in vivo distribution data of the radioactive microspheres and changes in the lesion site cannot be accurately obtained. Therefore, both yttrium [$^{90}$Y] resin microspheres and yttrium [$^{90}$Y] glass microspheres can only provide low-quality SPECT and PET images, which are difficult to provide significant and clear-image visualization functions for diagnosis and treatment. Once such radioactive microspheres have entered the lesion site, the following can only be assessed by other imaging techniques: 1. whether the agent has entered the target position accurately; 2. whether the radioactive microspheres have moved into other tissues and organs during the treatment; and 3. evaluation of the therapeutic effect for the treatment site. However, these imaging techniques usually only achieve short-term imaging, and a contrast agent and a therapeutic agent are two different agents or entities, and there are at least the following two problems: 1. the contrast agent needs to be administrated separately to achieve imaging, which brings great inconvenience to clinical use; and 2. the distribution and metabolic behavior of the contrast agent and the radioactive microspheres in the body are inconsistent, and the location of the radioactive microspheres cannot be accurately monitored for a long time.

Therefore, in order to realize the visualization of the entire administration process, accurately monitor the in vivo distribution of the radioactive therapeutic microspheres during the treatment process, and efficiently evaluate the therapeutic efficacy, the realization of high-quality visualization of the radioactive therapeutic microspheres is an issue which urgently needs to be addressed in the art. In the relevant state of the art, document CN 107 715 124 gives an example of simple carbon microspheres carrying 90 yttrium as therapeutic radionuclide. Document CN 107 715 122 also suggests carbon microspheres of medical yttrium phosphate $^{90}$Y$^{32}$PO$_4$ as a tumor radiotherapy medicament. The interest of radiolabeling for image and therapy has been suggested in document WO 2009/129577 A1. The latter suggests to complex a macromolecule composition with different FibrinLite compositions which are prepared with two different isotopes. G Ferro-Flores ET AL have also suggested carbon nanoparticles with an imaging and a therapeutic radionuclide in the article "Multifunctional Radiolabeled Nanoparticles for Targeted Therapy", Current Medicinal Chemistry, Vol. 21, no.1, (2014-01-01), pages 124-138.

## Summary of the Invention

In order to solve the problems in the background art and realize the visualized treatment of radioactive carbon microsphere products in the tumor treatment process, the present invention provides a visualized radioactive carbon microsphere suspension and a preparation method thereof. The carbon microsphere suspension has the characteristics of being clearly visible under PET and available for quantitative analysis, which enables visualization of the radiation therapy process in solid tumors without the aid of other contrast agents.

According to the technical solutions provided by the present invention, the visualized radioactive carbon microsphere suspension is radioactive carbon microspheres with high-energy beta-ray emission for solid tumor treatment and PET imaging, made by loading carbon microspheres having good biocompatibility with radioactive nuclides for treatment and zirconium [$^{89}$Zr] and dispersing into a special solution.

To achieve the above objectives, the first technical solution adopted by the present invention is:
a visualized radioactive carbon microsphere suspension, every 1 ml of the solution comprising: 10-500 mg of carbon microspheres, 5-500 mCi of activity of radioactive nuclides for treatment, 0.1-100 mCi of activity of radioactive nuclides for imaging, 0-100 mg of small organic molecules, and 0.1-1.0 ml of a first solution.

Further, the carbon microspheres are spherical or non-spherical carbon materials rich in micropores and mesopores prepared by any method, with a diameter of 0.05-1000 $\mu$m, preferably 20-60 $\mu$m. The visualized carbon micro-

sphere products prepared from carbon microspheres with different particle sizes can be used for visualized treatment of different solid tumor lesions due to different administration modes and different distributions in tissues, organs and lesions.

Further, the small organic molecule is 5-sulfosalicylic acid, 5-nitrosalicylic acid or a small molecule with a similar structure obtained by simple chemical modification.

Further, the radioactive nuclide for treatment is selected from any one of yttrium [$^{90}$Y], lutetium [$^{177}$Lu], holmium [$^{166}$Ho], samarium [$^{153}$Sm] and isotopes thereof, and the radioactive nuclide for imaging is zirconium [$^{89}$Zr],

Further, the first solution comprises, but is not limited to, an ethanol solution, a polyethylene glycol solution, and a glycerin solution; water-soluble carbohydrate solutions such as a glucose solution, a dextran solution, and a glucan solution; water-soluble cellulose solutions such as a sodium carboxymethyl cellulose solution, a sodium carboxyethyl cellulose solution, and a hydroxypropyl cellulose solution; water-soluble starch solutions such as a hydroxyethyl starch solution and a sodium carboxymethyl starch solution; and other water-soluble small-molecule or polymer solutions with similar structures.

Further, the radioactive nuclides for treatment and zirconium [$^{89}$Zr] are water-soluble radioactive nuclides for treatment and zirconium [$^{89}$Zr] in different chemical forms prepared by generators or nuclear reactors or any other common preparation methods. The radioactivity of the solution of radioactive nuclides for treatment is 10 mCi-100 Ci, and the radioactivity of the solution of radioactive zirconium [$^{89}$Zr] is 0.1 mCi-10 Ci.

The second technical solution adopted by the present invention is:

a method for preparing the visualized radioactive carbon microsphere suspension, the method comprising the following steps:

a solution of radioactive nuclides for treatment is uniformly mixed with an aqueous solution of small organic molecules having a first pH value;

the radioactive nuclides for treatment in the above mixed solution is adsorbed by using carbon microspheres;

the carbon microspheres after adsorption of the radioactive nuclides for treatment are mixed and reacted with a sodium phosphate solution, followed by washing and solid-liquid separation, to obtain a first intermediate;

the first intermediate is uniformly mixed with an aqueous solution of small organic molecules having a first pH value to obtain a first intermediate solution;

an ionic solution of radioactive zirconium [$^{89}$Zr] having a second pH value is added to the first intermediate solution to obtain a second intermediate; and

the first solution is added to the second intermediate, and they are mixed uniformly and sterilized.

The third technical solution adopted by the present invention is:

a method for preparing the visualized radioactive carbon microsphere suspension, the method comprising the following steps:

small organic molecules in an aqueous solution of small organic molecules having a first pH value are adsorbed by using carbon microspheres;

radioactive zirconium [$^{89}$Zr] is adsorbed by using the carbon microspheres after adsorption of small organic molecules to obtain a third intermediate;

the radioactive nuclides for treatment in the mixed solution of the ions of radioactive nuclides for treatment and the aqueous solution of small organic molecules are adsorbed by using the third intermediate;

the third intermediate after adsorption of the radioactive nuclides for treatment is mixed and reacted with a sodium phosphate solution, followed by washing and solid-liquid separation, to obtain a fourth intermediate; and

the first solution is added to the fourth intermediate, and they are mixed uniformly and sterilized.

Further, the first pH value is 1-14, preferably 3.5-6.5; and the second pH value is 1-14, preferably 3.5-7.5.

The present invention also provides a use of the aforementioned visualized radioactive carbon microsphere suspension for preparing an agent for visualized tumor treatment, wherein the tumor comprises, but is not limited to solid tumors including liver cancer, pancreatic cancer, renal cancer, breast cancer, thyroid cancer and intestinal cancer, and orthopedic tumors.

Further, the visualized tumor treatment is to simultaneously realize local radioactive treatment and real-time imaging of tumor lesions, and the imaging means is PET/CT.

The present invention is made by with carbon microspheres having good biocompatibility as carriers, dispersing carbon microspheres loaded with radioactive nuclides for treatment and zirconium [$^{89}$Zr] into a special solution, can realize uniform distribution in tumor tissues through catheter intervention, injection and other administration means, and can carry out tumor implantation for various solid tumors, including liver cancer, pancreatic cancer, kidney cancer, breast cancer, thyroid cancer, intestinal cancer and other solid tumors, to achieve visualized brachytherapy.

The visualized carbon microspheres of the present invention are mainly used for the treatment of solid tumors and visualized treatment by positron emission tomography-computed tomography (PET/CT) during the treatment process, and are simultaneously used as an agent for treatment and an agent for imaging. The visualized carbon microspheres can enter into tumors by means of injection, intervention, etc., to achieve the killing of tumor cells by emitting high-energy beta rays by radioactive nuclides for treatment, and to achieve imaging by collecting positrons emitted by zirconium [$^{89}$Zr] by PET, thereby realizing the visualized treatment of tumor lesions.

Compared with the prior art, the present invention has the beneficial effects that:

1. Radioactive nuclides for treatment and nuclides for imaging in different groups in the periodic table of elements usually have completely different chemical properties, and it is difficult to achieve stable and efficient loading on the same carrier. The preparation method of the present invention overcomes this technical problem, and achieves simultaneous loading on carbon microspheres of radioactive nuclides for treatment (any of yttrium [$^{90}$Y], lutetium [$^{177}$Lu], holmium [$^{166}$Ho], samarium [$^{153}$Sm] and isotopes thereof) and nuclides for imaging, zirconium [$^{89}$Zr], and the radioactive nuclides in the prepared carbon microsphere products have a loading rate of more than 98% and a release rate of less than 0.1%.

2. The visualized radioactive carbon microsphere suspension of the present invention can achieve local radioactive treatment and real-time imaging for solid tumor lesions at the same time to achieve visualized treatment of tumors; a novel radioactive carbon microsphere product integrating diagnosis and treatment is provided; and the visualized carbon microsphere of the present invention is a brand-new radioactive carbon microsphere product loaded with both the radioactive nuclides for treatment and the nuclide for imaging, zirconium [$^{89}$Zr], which has not been found in other documents and patents.

3. The visualized carbon microspheres of the present invention exhibit significant tumor treatment effects, and can provide high-quality PET/CT images during the treatment process; and the microspheres have the potential to be developed as a therapeutic agent in solid tumors such as liver cancer, pancreatic cancer, kidney cancer, breast cancer, thyroid cancer, and intestinal cancer.

## Brief Description of the Drawings

Fig. 1 is the SPECT images after single hepatic arterial administration of yttrium [$^{90}$Y] carbon microspheres in Beagle dogs in the background art;

Fig. 2 is the scanning electron micrographs of visualized radioactive carbon microspheres in Example 6;

Fig. 3 is the images of local tumor anatomy after 15 days of treatment on New Zealand rabbits in an experimental group in Example 11; and

Fig. 4 is the MIP images of PET/CT scanning at different time points after a single hepatic arterial administration of visualized carbon microspheres on New Zealand rabbits in Example 12.

## Detailed Description of Embodiments

In order to better understand the technical solutions of the present invention, the technical solutions of the present invention will be further described below with reference to the accompanying drawings and examples. Modes for implementing the present invention include, but are not limited to, the following examples, which are used to illustrate the present invention, and are not intended to limit the protection scope of the present invention. Unless otherwise specified, the technical means used in the examples are conventional means well known to those skilled in the art. The test methods in the following examples are conventional methods unless otherwise specified.

The methods for determining the labeling rate and release rate of a radioactive nuclide used in the examples of the present invention are as follows:

(1) a visualized radioactive carbon microsphere suspension in an example is taken, and an isotope activity measuring instrument (CRC-25R) is used to measure the total activity of the product ($A_{carbon\ microsphere\ suspension}$ or $A_{(0)}$);

(2) 100 $\mu$L of the supernatant of the above suspension product is taken, and quantitative determination is performed by using a high-purity germanium gamma spectrometer (GEM-C40-LB-C) to obtain the activity value of the supernatant ($A_{supernatant}$); and

(3) the remaining microsphere solids in step (2) are placed into a centrifuge tube, and carbon microsphere solids are obtained after centrifugation. The carbon microsphere solids are soaked in 10 ml of a sodium chloride injection with a concentration of 0.9% by mass, and they are placed into a constant temperature oscillator at 37 $\pm$ 1°C. 1 ml of the supernatant after shaking for 24 h is taken and filtered. 100 $\mu$L of the filtered liquid is taken, quantitative determination is performed by using a high-purity germanium gamma spectrometer (GEM-C40-LB-C) to obtain the activity value $A_{(ti)}$, and the release rate of radioactive nuclides from the visualized radioactive carbon microspheres at different oscillation times is calculated.

(a) The adsorption rate (i.e., labeling rate) of radioactive nuclides by carbon microspheres is calculated according to formula (1):

$$\text{Adsorption rate} = 1 - \frac{A_{\text{Supernatant}}}{A_{\text{Carbon microsphere suspension}} \times k} \quad \cdots\cdots\cdots\cdots (1)$$

in which formula, $A_{\text{supernatant}}$ - the activity of the supernatant after the carbon microspheres are adsorbed with radioactive nuclides;
$A_{\text{carbon microsphere suspension}}$ - the activity of radioactive nuclides in the carbon microsphere suspension (normalized to the measurement point of the adsorption supernatant);
k - calibration factor for the activity meter to measure the radioactive nuclide under the corresponding geometric conditions.
(b) At different points, the release rate of radioactive nuclides from the carbon microspheres is calculated according to the formula (2):

$$\text{Release rate} = \frac{A_{(t_i)}}{A_{(0)}e^{-\lambda t}} \quad \cdots\cdots\cdots\cdots\cdots (2)$$

in which formula: t - time interval from point t=0 to the measurement point;
$A_{(ti)}$ - activity of radioactive nuclide in the soaking solution at t point;
A(0) - activity of radioactive nuclide in the visualized radioactive nuclide carbon microsphere suspension at point t=0.

Considering the correction of each sampling loss, the total activity of radioactive nuclides $A(t_i)$ of the soaking solution measured at the i-th time is calculated according to the formula (3):

$$A(t_i) = V \times a_i + \sum_{j=2}^{i} a_{j-1} e^{-\lambda(t_i - t_{j-1})} \quad \cdots\cdots\cdots\cdots\cdots\cdots\cdots\cdots\cdots (3)$$

in which formula: V - total volume of soaking supernatant;
$a_i$, $a_j$ - radioactive concentrations of the soaking solution measured at the i-th and j-th times, respectively; and
$t_i$, $t_j$ - soaking times of the i-th sampling and j-th sampling, respectively.

**Example 1**

A visualized radioactive carbon microsphere suspension, every 1 ml of the solution comprising: 10-500 mg of carbon microspheres, 5-500 mCi of activity of radioactive nuclides for treatment, 0.1-100 mCi of activity of radioactive zirconium [89Zr], 0-100 mg of small organic molecules, and 0.1-1.0 ml of a first solution.
Specifically, the carbon microspheres are spherical or non-spherical carbon materials rich in micropores and mesopores prepared by any method, with a diameter of 0.05-1000 $\mu$m, preferably 20-60 $\mu$m. The visualized carbon microsphere products prepared from carbon microspheres with different particle sizes can be used for visualized treatment of different solid tumor lesions due to different administration modes and different distributions in tissues, organs and lesions.
Specifically, the small organic molecule is 5-sulfosalicylic acid, 5-nitrosalicylic acid or a small molecule with a similar structure obtained by simple chemical modification.
Specifically, the first solution comprises, but is not limited to, an ethanol solution, a polyethylene glycol solution, and a glycerin solution; water-soluble carbohydrate solutions such as a glucose solution, a dextran solution, and a glucan solution; water-soluble cellulose solutions such as a sodium carboxymethyl cellulose solution, a sodium carboxyethyl cellulose solution, and a hydroxypropyl cellulose solution; water-soluble starch solutions such as a hydroxyethyl starch solution and a sodium carboxymethyl starch solution; and one or more of other water-soluble small-molecule or polymer solutions with similar structures.

Specifically, the radioactive nuclide for treatment is any one of yttrium [$^{90}$Y], lutetium [$^{177}$Lu], holmium [$^{166}$Ho], samarium [$^{153}$Sm] and isotopes thereof. The radioactive nuclides for treatment and zirconium [$^{89}$Zr] both are water-soluble radioactive nuclides for treatment and zirconium [$^{89}$Zr] in different chemical forms prepared by generators or nuclear reactors or any other common preparation methods. The radioactivity of the solution of radioactive nuclides for treatment is 10 mCi-100 Ci, and the radioactivity of the solution of radioactive zirconium [$^{89}$Zr] for imaging is 0.1 mCi-10 Ci.

**Example 2**

A method for preparing the visualized radioactive carbon microsphere suspension, the method comprising the following steps:

a solution of radioactive nuclides for treatment was uniformly mixed with an aqueous solution of small molecules and then it was allowed to stand for 2 min-20 min;

the radioactive nuclides for treatment in the above mixed solution was adsorbed by using carbon microspheres and it was allowed to stand for 2 min-20 min;

the carbon microspheres after adsorption of the radioactive nuclides for treatment were mixed and reacted with a sodium phosphate solution and it was allowed to stand for 2 min-20 min, followed by washing and solid-liquid separation, to obtain a first intermediate;

the first intermediate was uniformly mixed with an aqueous solution of small organic molecules having a pH value of 1-14, preferably 3.5-6.5, and then it was allowed to stand for 2 min-20 min to obtain a first intermediate solution;

an ionic solution of radioactive zirconium [$^{89}$Zr] having a pH value of 1-14, preferably 3.5-7.5 was added to the first intermediate solution, and they were uniformly mixed and then allowed to stand for 2 min-20 min to obtain a second intermediate; and

the first solution was added to the second intermediate, and they were mixed uniformly, dispensed and subjected to moist heat sterilization, where the sterilization conditions were 121°C, 15 min.

**Example 3**

A method for preparing the visualized radioactive carbon microsphere suspension, the method comprising the following steps:

small organic molecules in an aqueous solution of small organic molecules having a pH value of 1-14, preferably 3.5-6.5 were adsorbed by using carbon microspheres;

radioactive zirconium [$^{89}$Zr] was adsorbed by using the carbon microspheres after adsorption of small organic molecules to obtain a third intermediate;

the radioactive nuclides for treatment in the mixed solution of radioactive nuclides for treatment and the aqueous solution of small organic molecules were adsorbed by using the third intermediate;

the third intermediate after adsorption of the radioactive nuclides for treatment was mixed and reacted with a sodium phosphate solution, followed by washing and solid-liquid separation, to obtain a fourth intermediate; and

the first solution was added to the fourth intermediate, and they were mixed uniformly, dispensed and subjected to moist heat sterilization, where the sterilization conditions were 121°C, 15 min.

**Example 4**

Use of a visualized radioactive carbon microsphere suspension for preparing an agent for visualized treatment of solid tumors. The present invention is made by with carbon microspheres having good biocompatibility as carriers, dispersing carbon microspheres loaded with radioactive nuclides for treatment and the nuclide for imaging, zirconium [$^{89}$Zr] into a special solution, can realize uniform distribution in tumor tissues through catheter intervention, injection and other administration means, and can carry out tumor implantation for various solid tumors, including liver cancer, pancreatic cancer, kidney cancer, breast cancer, thyroid cancer, intestinal cancer and other solid tumors, to achieve visualized brachytherapy.

**Example 5**

Use of a visualized radioactive carbon microsphere suspension for preparing an agent for PET/CT. The visualized carbon microspheres of the present invention are mainly used for the treatment of solid tumors and visualized

**EP 4 112 085 B1**

treatment by positron emission tomography-computed tomography (PET/CT) during the treatment process, and are simultaneously used as an agent for treatment and an agent for imaging. The visualized carbon microspheres can enter into tumors by means of injection, intervention, etc., to achieve the killing of tumor cells by emitting high-energy beta rays by radioactive nuclides for treatment, and to achieve imaging by collecting positrons emitted by zirconium [$^{89}$Zr] by PET, thereby realizing the visualized treatment of tumor lesions.

**Example 6**

A visualized radioactive carbon microsphere suspension, the preparation method of which comprising:

A radioactive yttrium [$^{90}$Y] solution containing yttrium [$^{89}$Y] and a 5-sulfosalicylic acid solution at pH 4.5 were mixed in a molar ratio of yttrium to 5-sulfosalicylic acid 1 : (1-100), shaken uniformly and allowed to stand for 2-20 min;
0.01-100 g of carbon microspheres were used to adsorb the radioactive yttrium [$^{90}$Y] in the above mixed solution, and they were shaken uniformly and allowed to stand for 2-20 min;
the carbon microspheres after adsorption of the radioactive yttrium [$^{90}$Y] were mixed and reacted with a sodium phosphate solution at pH 6.5 with a molar concentration of 1%, followed by washing and solid-liquid separation to obtain a first intermediate after separation;
the first intermediate and an aqueous solution containing 0.01-10 mg of 5-sulfosalicylic acid at pH 4.5 were shaken uniformly and allowed to stand for 2-20 min to obtain a first intermediate solution;
a zirconium [$^{89}$Zr] solution at pH 4.0 was added to the first intermediate solution, and they were shaken uniformly and allowed to stand for 2-20 min to obtain a second intermediate; the supernatant was taken for measurement of the radioactivity of zirconium [$^{89}$Zr], and the adsorption rate of radioactive nuclides was calculated to be 99.1%.

1-100 ml of a sodium carboxymethyl cellulose solution with a molar concentration of 0.2% was added to the second intermediate, and they were mixed uniformly, dispensed into vials, and sterilized under the conditions of 121°C and 15 min to obtain a visualized carbon microsphere suspension available for injection.
The adsorption rate of the radioactive nuclides in the sample of this example is 99.6%, and the release rate is 0.006%.
The scanning electron micrographs of the prepared visualized radioactive carbon microspheres are shown in Fig. 2. The particle size of the microspheres is mainly 20-45 μm, and the surface is smooth.

**Example 7**

A visualized radioactive carbon microsphere suspension, the preparation method of which comprising:

0.01-100 g of carbon microspheres were used to adsorb 5-sulfosalicylic acid in an aqueous solution containing 0.01-10 mg of 5-sulfosalicylic acid at pH 4.0, and they were shaken uniformly and allowed to stand for 2-20 min;
the above-mentioned carbon microspheres adsorbed with 5-sulfosalicylic acid were added to 0.1-10 Ci of a zirconium [$^{89}$Zr] solution at pH 5.0, and they were shaken uniformly and allowed to stand for 2-20 min to obtain a third intermediate; the supernatant was taken for measurement of the radioactivity of zirconium [$^{89}$Zr], and the adsorption rate of radioactive nuclides was calculated to be 99.3%; a radioactive yttrium [$^{90}$Y] solution containing yttrium [$^{89}$Y] and a 5-sulfosalicylic acid solution were mixed in a molar ratio of yttrium to 5-sulfosalicylic acid 1 : (1-100), shaken uniformly and allowed to stand for 2-20 min;
the third intermediate was used to adsorb the radioactive yttrium [$^{90}$Y] in the above-mentioned mixed solution of radioactive yttrium [$^{90}$Y] and 5-sulfosalicylic acid, and they were shaken uniformly and allowed to stand for 2-20 min;
the third intermediate after adsorption of the radioactive yttrium [$^{90}$Y] were mixed and reacted with a sodium phosphate solution at pH 6.5 with a molar concentration of 1%, followed by washing and solid-liquid separation to obtain a fourth intermediate;
1-100 ml of a hydroxyethyl starch solution with a molar concentration of 6% was added to the fourth intermediate, and they were mixed uniformly, dispensed into vials, and sterilized under the conditions of 121°C and 15 min to obtain a visualized carbon microsphere suspension available for injection.

The adsorption rate of the radioactive nuclides in the sample of this example is 99.8%, and the release rate is 0.023%.

**Example 8**

A visualized radioactive carbon microsphere suspension, the preparation method of which comprising:

A radioactive solution containing 400 mCi of lutetium[175Lu] and lutetium[177Lu] and a 5-sulfosalicylic acid solution at pH 3.5 were mixed in a molar ratio of lutetium to 5-sulfosalicylic acid 1 : 4, shaken uniformly and allowed to stand for 10 min;

1.0 g of carbon microspheres were used to adsorb the radioactive lutetium[175Lu] and lutetium[177Lu] in the above mixed solution, and they were shaken uniformly and allowed to stand for 2-20 min; the supernatant was taken for measurement of the radioactivity of lutetium[177Lu], and the adsorption rate of radioactive nuclides was calculated to be 98.8%; the carbon microspheres after adsorption of the radioactive lutetium[175Lu] and lutetium[177Lu] were mixed and reacted with a sodium phosphate solution at pH 6.5 with a molar concentration of 1%, followed by washing and solid-liquid separation to obtain a first intermediate after separation;

the first intermediate and an aqueous solution containing 3 mg of 5-sulfosalicylic acid at pH 3.5 were shaken uniformly and allowed to stand for 2 min to obtain a first intermediate solution;

10 mCi of a zirconium [89Zr] solution at pH 4.5 was added to the first intermediate solution, and they were shaken uniformly and allowed to stand for 20 min to obtain a second intermediate;

5 ml of a sodium carboxymethyl cellulose solution with a molar concentration of 0.2% was added to the second intermediate, and they were mixed uniformly, dispensed into 20 vials, and sterilized under the conditions of 121°C and 15 min to obtain a visualized carbon microsphere suspension available for injection.

The adsorption rate of the radioactive nuclides in the sample of this example is 99.9%, and the release rate is 0.056%.

## Example 9

A visualized radioactive carbon microsphere suspension, the preparation method of which comprising:

A radioactive solution containing 800 mCi of holmium[165Ho] and holmium[166Ho] and a 5-sulfosalicylic acid solution at pH 6.5 were mixed in a molar ratio of holmium to 5-sulfosalicylic acid 1 : 3, shaken uniformly and allowed to stand for 5 min;

1.0 g of carbon microspheres were used to adsorb the radioactive holmium[165Ho] and holmium[166Ho] in the above mixed solution, and they were shaken uniformly and allowed to stand for 5 min;

the carbon microspheres after adsorption of the radioactive holmium[165Ho] and holmium[166Ho] were mixed and reacted with a sodium phosphate solution at pH 6.5 with a molar concentration of 1%, followed by washing and solid-liquid separation to obtain a first intermediate after separation;

the first intermediate and an aqueous solution containing 1 mg of 5-sulfosalicylic acid at pH 6.5 were shaken uniformly and allowed to stand for 6 min to obtain a first intermediate solution;

5 mCi of a zirconium [89Zr] solution at pH 4.5 was added to the first intermediate solution, and they were shaken uniformly and allowed to stand for 20 min to obtain a second intermediate; the supernatant was taken for measurement of the radioactivity of zirconium [89Zr], and the adsorption rate of radioactive nuclides was calculated to be 99.8%;

5 ml of a sodium carboxymethyl cellulose solution with a molar concentration of 0.2% was added to the second intermediate, and they were mixed uniformly, dispensed into 20 vials, and sterilized under the conditions of 121°C and 15 min to obtain a visualized carbon microsphere suspension available for injection.

The adsorption rate of the radioactive nuclides in the sample of this example is 99.5%, and the release rate is 0.034%.

## Example 10

A visualized radioactive carbon microsphere suspension, the preparation method of which comprising:

A radioactive solution containing 1000 mCi of samarium[150Sm] and samarium[153Sm] and a 5-sulfosalicylic acid solution at pH 5.5 were mixed in a molar ratio of samarium to 5-sulfosalicylic acid 1 : 8, shaken uniformly and allowed to stand for 15 min;

1.0 g of carbon microspheres were used to adsorb the radioactive samarium[150Sm] and samarium[153Sm] in the above mixed solution, and they were shaken uniformly and allowed to stand for 5 min;

the carbon microspheres after adsorption of the radioactive samarium[150Sm] and samarium[153Sm] were mixed and reacted with a sodium phosphate solution at pH 6.5 with a molar concentration of 1%, followed by washing and solid-liquid separation to obtain a first intermediate after separation;

the first intermediate and an aqueous solution containing 1 mg of 5-sulfosalicylic acid at pH 5.5 were shaken uniformly and allowed to stand for 6 min to obtain a first intermediate solution;

8 mCi of a zirconium [89Zr] solution at pH 4.5 was added to the first intermediate solution, and they were shaken uniformly and allowed to stand for 20 min to obtain a second intermediate; the supernatant was taken for measurement of the radioactivity of zirconium [89Zr], and the adsorption rate of radioactive nuclides was calculated to be 99.9%; 5 ml of a sodium carboxymethyl cellulose solution with a molar concentration of 0.2% was added to the second intermediate, and they were mixed uniformly, dispensed into 20 vials, and sterilized under the conditions of 121°C and 15 min to obtain a visualized carbon microsphere suspension available for injection.

The adsorption rate of the radioactive nuclides in the sample of this example is 99.3%, and the release rate is 0.018%.

**Example 11**

The efficacy evaluation was carried out by in situ tumor injection of the visualized carbon microsphere suspension of Example 7 of the present application in an xenograft model on the body surface of New Zealand rabbits:

experimental group: 4 New Zealand rabbits; dosage is: 50-100 mg of visualized carbon microsphere suspension/rabbit, where the activity with yttrium [89Y] is 1.3-2.5 mCi, and the activity with zirconium [89Zr] is 0.13-0.25 mCi; average dosage per rabbit: 80 mg; administration volume: 5% of tumor volume;
control group: 2 tumor-bearing New Zealand rabbits; given with an equal volume of normal saline.

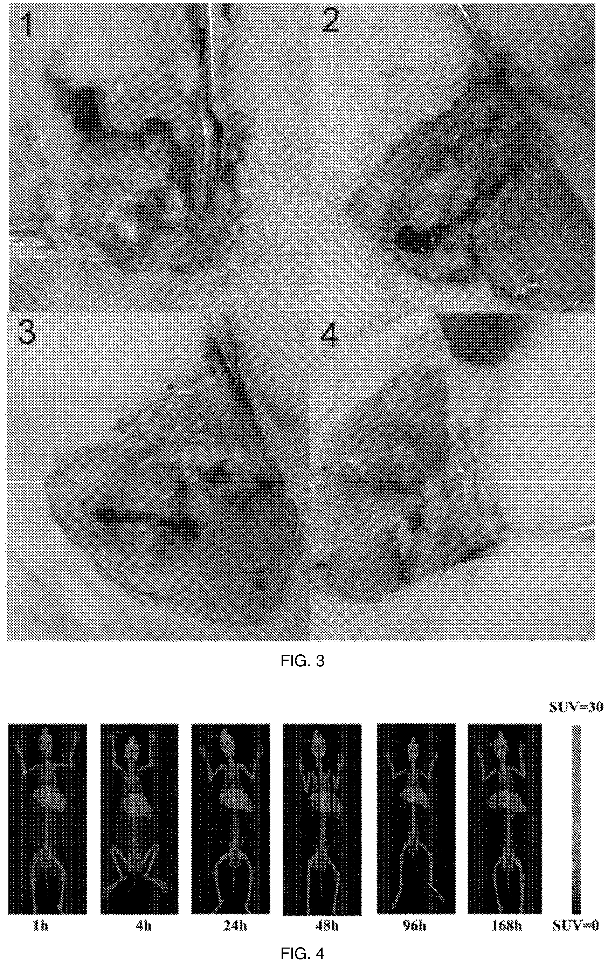

Ultrasound observation and size measurement after 5 days of treatment showed that the tumors of 4 New Zealand rabbits in the experimental group did not grow, while the tumors of 2 New Zealand rabbits in the control group increased significantly in volume. Local anatomy of the New Zealand rabbits was performed after 15 days of treatment. The local anatomy of 4 rabbits in the experimental group is shown in Fig. 3. Through anatomy, it is obviously observed that black visualized carbon microspheres are locally visible in the tumor tissues of the 4 New Zealand rabbits at the administration site, and it can be seen that the tumor tissues near the visualized carbon microspheres become grayish brown, with obvious fibrosis and necrosis. It is proved that the visualized carbon microsphere suspension prepared in the examples of the present application exhibits good antitumor activity.

**Example 12**

The visualized carbon microsphere suspension of Example 7 of the present application was administered to New Zealand rabbits through hepatic artery catheterization surgery, and the distribution of the visualized carbon microsphere suspension in animals was studied: The dosage of a single New Zealand rabbit was about 20 mg, and PET scans on whole body of the rabbits in different beds was performed at six time points of 1h, 4h, 24h, 48h, 96h and 168h after administration of the visualized radioactive carbon microsphere suspension, and while maintaining the animals stationary, CT scans were done before/after PET scans. The results are shown in Fig. 4.

After the PET/CT scans were completed, image reconstruction was performed, and PMOD software was used to process the images and data. Organs such as brain, heart, liver, spleen, lungs, kidneys, stomach, bones, and muscles were outlined as regions of interest, and the radioactivity concentrations (namely, radioactivity value per unit volume) in the regions of interest were obtained. Then, the decay correction was performed on the activity at each time point. The results are shown in Table 1. The formulas used are as follows:

$$\text{Corrected activity} = \text{Initial activity} \times 0.5^{\wedge} \frac{(\text{Correction time point} - \text{Initial time point})}{\text{Physical half - Life of nuclide}}$$

$$\text{Radioactive concentration of tissue} \, (\mu g \, \text{Equ./g}) = \frac{\text{Radioactivity concentration of region of interest} \, (\mu Ci/g)}{\text{Specific activity} \, (\mu Ci/\mu g)}$$

Table 1 Radioactive concentrations of each tissue at different time points after a single hepatic arterial administration of visualized carbon microspheres in New Zealand rabbits

| Time point/h Tissue | 1 | 4 | 24 | 48 | 96 | 168 |
|---|---|---|---|---|---|---|
| Brain | 0.02±0.01 | 0.04±0.03 | 0.13±0.03 | 0.14±0.02 | 0.19±0.11 | 0.11±0.01 |
| Heart | 0.14±0.02 | 0.17±0.05 | 0.10±0.07 | 0.16±0.01 | 0.10±0.02 | 0.09±0.01 |
| Left lung | 0.42±0.26 | 0.50±0.39 | 0.40±0.33 | 1.45±0.73 | 1.14±0.79 | 0.47±0.34 |
| Right lung | 0.46±0.13 | 0.25±0.06 | 0.35±0.05 | 2.21±0.24 | 0.65±0.14 | 0.29±0.03 |
| Liver left | 90.49±21.72 | 82.38±42.46 | 89.49±64.34 | 92.15±61.28 | 100.90±65.23 | 62.62±32.14 |
| Liver right | 126.29±37.63 | 109.20±66.47 | 110.38±75.38 | 121.40±76.37 | 100.08±46.76 | 98.50±49.47 |
| Kidney | 0.12±0.04 | 0.13±0.00 | 0.18±0.11 | 0.24±0.10 | 0.15±0.09 | 0.19±0.05 |
| Spleen | 0.05±0.03 | 0.08±0.08 | 0.07±0.06 | 0.13±0.00 | 0.08±0.05 | 0.10±0.10 |
| Bone joint | 0.00±0.00 | 0.04±0.06 | 0.52±0.26 | 1.03±0.18 | 1.04±0.02 | 0.96±0.31 |
| Tibia | 0.00±0.00 | 0.00±0.00 | 0.00±0.01 | 0.01±0.01 | 0.02±0.00 | 0.01±0.00 |
| Muscle | 0.00±0.00 | 0.01±0.01 | 0.01±0.00 | 0.01±0.00 | 0.01±0.01 | 0.02±0.00 |
| Stomach | 0.15±0.09 | 0.20±0.05 | 0.20±0.08 | 0.25±0.15 | 0.20±0.19 | 0.33±0.22 |

A clear distribution image of visualized carbon microspheres in animals is obtained in Fig. 3. Combining with the data on uptake in each organ and tissue in Table 1, it can be seen that the visualized carbon microspheres are concentrated in the liver, accounting for more than 99%, with small uptake in other organs and tissues.

From the results of Fig. 2, Fig. 3, Fig. 4 and Table 1, it can be seen that: the loading rate of radioactive nuclides in the carbon microsphere products prepared by the present invention is greater than 98%, and the release rate is less than 0.1%; and local radioactive treatment and real-time imaging for solid tumor lesions are achieved at the same time, that is, visualized treatment of tumors is achieved.

## Claims

1. A visualized radioactive carbon microsphere suspension, every 1 ml of the solution comprising: 10-500 mg of carbon microspheres, 5-500 mCi of activity of radioactive nuclides for treatment, 0.1-100 mCi of activity of radioactive nuclides for imaging, 0-100 mg of small organic molecules, and 0.1-1.0 ml of a first solution, and wherein the radioactive nuclide for treatment is selected from any one of yttrium [$^{90}$Y], lutetium [$^{177}$Lu], holmium [$^{166}$Ho], samarium [$^{153}$Sm] and isotopes thereof, the radioactive nuclide for imaging is zirconium [$^{89}$Zr], and the small organic molecules is 5-sulfosalicylic acid or 5-nitrosalicylic acid.

2. The suspension of claim 1, wherein the carbon microspheres are spherical or non-spherical carbon materials rich in micropores and mesopores prepared by any method, with a diameter of 0.05-1000 $\mu$m, preferably 20-60 $\mu$m.

3. The suspension of claim 1, wherein the first solution comprises, but is not limited to, an ethanol solution, a polyethylene glycol solution, and a glycerin solution; water-soluble carbohydrate solutions such as a glucose solution, a dextran solution, and a glucan solution; water-soluble cellulose solutions such as a sodium carboxymethyl cellulose solution, a sodium carboxyethyl cellulose solution, and a hydroxypropyl cellulose solution; water-soluble starch solutions such as a hydroxyethyl starch solution and a sodium carboxymethyl starch solution; and other water-soluble small-molecule or polymer solutions with similar structures.

4. A method for preparing the visualized radioactive carbon microsphere suspension of any one of claims 1-3, the method comprising the following steps:

a solution of radioactive nuclides for treatment is uniformly mixed with an aqueous solution of small organic molecules having a first pH value, wherein the radioactive nuclide for treatment is selected from any one of yttrium [$^{90}$Y], lutetium [$^{177}$Lu], holmium [$^{166}$Ho], samarium [$^{153}$Sm] and isotopes thereof, and the small organic molecules is 5-sulfosalicylic acid or 5-nitrosalicylic acid;

the radioactive nuclides for treatment in the above mixed solution is adsorbed by using carbon microspheres;

the carbon microspheres after adsorption of the radioactive nuclides for treatment are mixed and reacted with a sodium phosphate solution, followed by washing and solid-liquid separation, to obtain a first intermediate;

the first intermediate is uniformly mixed with an aqueous solution of small organic molecules having a first pH value to obtain a first intermediate solution and wherein the small organic molecules is 5-sulfosalicylic acid or 5-nitrosalicylic acid;

an ionic solution of radioactive zirconium [$^{89}$Zr] having a second pH value is added to the first intermediate solution to obtain a second intermediate; and

the first solution is added to the second intermediate, and they are mixed uniformly and sterilized.

5. A method for preparing the visualized radioactive carbon microsphere suspension of any one of claims 1-3, the method comprising the following steps:

small organic molecules in an aqueous solution of small organic molecules having a first pH value are adsorbed by using carbon microspheres and wherein the small organic molecules is 5-sulfosalicylic acid or 5-nitrosalicylic acid;

radioactive zirconium [$^{89}$Zr] is adsorbed by using the carbon microspheres after adsorption of small organic molecules to obtain a third intermediate;

the radioactive nuclides for treatment in the mixed solution of the ions of radioactive nuclides for treatment and the aqueous solution of small organic molecules are adsorbed by using the third intermediate;

the third intermediate after adsorption of the radioactive nuclides for treatment is mixed and reacted with a sodium phosphate solution, followed by washing and solid-liquid separation, to obtain a fourth intermediate; and

the first solution is added to the fourth intermediate, and they are mixed uniformly and sterilized.

6. The method of claim 4 or 5, wherein the first pH value is 1-14, preferably 3.5-6.5; and the second pH value is 1-14, preferably 3.5-7.5.

7. A visualized radioactive carbon microsphere suspension prepared by the method of any one of claims 4-6.

8. A use of the visualized radioactive carbon microsphere suspension of any one of claims 1-3 and 7 for preparing an agent for visualized tumor treatment, wherein the tumor comprises, but is not limited to solid tumors including liver cancer, pancreatic cancer, renal cancer, breast cancer, thyroid cancer and intestinal cancer, and orthopedic tumors.

**Patentansprüche**

1. Sichtbar gemachte radioaktive Kohlenstoffmikrokugelsuspension, wobei die Lösung je 1 ml Folgendes umfasst: 10-500 mg Kohlenstoffmikrokugeln, 5-500 mCi Aktivität radioaktiver Nuklide zur Behandlung, 0,1-100 mCi Aktivität radioaktiver Nuklide zur Bildgebung, 0-100 mg kleiner organischer Moleküle und 0,1-1,0 ml einer ersten Lösung, und wobei das radioaktive Nuklid zur Behandlung aus Yttrium [$^{90}$Y], Lutetium [$^{177}$Lu], Holmium [$^{166}$Ho], Samarium [$^{153}$Sm] und Isotopen davon ausgewählt ist, wobei das radioaktive Nuklid für die Bildgebung Zirkonium [$^{89}$Zr] ist und die kleinen organischen Moleküle 5-Sulfosalicylsäure oder 5-Nitrosalicylsäure sind.

2. Suspension nach Anspruch 1, wobei die Kohlenstoffmikrokugeln kugelförmige oder nicht kugelförmige Kohlenstoffmaterialien sind, die einen hohen Anteil an Mikroporen und Mesoporen, die nach einem beliebigen Verfahren hergestellt wurden, mit einem Durchmesser von 0,05-1000 $\mu$m, vorzugsweise 20-60 um, aufweisen.

3. Suspension nach Anspruch 1, wobei die erste Lösung unter anderem eine Ethanollösung, eine Polyethylenglykollösung und eine Glycerinlösung; wasserlösliche Kohlenhydratlösungen wie eine Glucoselösung, eine Dextranlösung und eine Glucanlösung; wasserlösliche Celluloselösungen wie eine Natriumcarboxymethylcelluloselösung, eine Natriumcarboxyethylcelluloselösung und eine Hydroxypropylcelluloselösung; wasserlösliche Stärkelösungen wie eine Hydroxyethylstärkelösung und eine Natriumcarboxymethylstärkelösung; und andere wasserlösliche Lösungen kleiner Moleküle oder Polymere mit ähnlichen Strukturen umfasst.

4. Verfahren zum Herstellen der sichtbar gemachten radioaktiven Kohlenstoffmikrokugelsuspension nach einem der Ansprüche 1-3, wobei das Verfahren die folgenden Schritte umfasst:

eine Lösung radioaktiver Nuklide zur Behandlung wird gleichmäßig mit einer wässrigen Lösung kleiner organischer Moleküle mit einem ersten pH-Wert gemischt, wobei das radioaktive Nuklid zur Behandlung aus Yttrium [$^{90}$Y], Lutetium [$^{177}$Lu], Holmium [$^{166}$Ho], Samarium [$^{153}$Sm] und Isotopen davon ausgewählt ist, und die kleinen organischen Moleküle 5-Sulfosalicylsäure oder 5-Nitrosalicylsäure sind;
die radioaktiven Nuklide zur Behandlung in der oben genannten gemischten Lösung werden unter Verwendung von Kohlenstoffmikrokugeln adsorbiert;
die Kohlenstoffmikrokugeln werden nach der Adsorption der radioaktiven Nuklide zur Behandlung gemischt und mit einer Natriumphosphatlösung umgesetzt, gefolgt von Waschen und Fest-Flüssig-Abscheidung, um ein erstes Zwischenprodukt zu erhalten;
das erste Zwischenprodukt wird gleichmäßig mit einer wässrigen Lösung kleiner organischer Moleküle mit einem ersten pH-Wert gemischt, um eine erste Zwischenlösung zu erhalten, und wobei die kleinen organischen Moleküle 5-Sulfosalicylsäure oder 5-Nitrosalicylsäure sind;
eine ionische Lösung von radioaktivem Zirkonium [$^{89}$Zr] mit einem zweiten pH-Wert wird zur ersten Zwischenlösung hinzugefügt, um ein zweites Zwischenprodukt zu erhalten; und
die erste Lösung wird zum zweiten Zwischenprodukt hinzugefügt, und sie werden gleichmäßig gemischt und sterilisiert.

5. Verfahren zum Herstellen der sichtbar gemachten radioaktiven Kohlenstoffmikrokugelsuspension nach einem der Ansprüche 1-3, wobei das Verfahren die folgenden Schritte umfasst:

kleine organische Moleküle in einer wässrigen Lösung kleiner organischer Moleküle mit einem ersten pH-Wert werden unter Verwendung von Kohlenstoffmikrokugeln adsorbiert, und wobei die kleinen organischen Moleküle 5-Sulfosalicylsäure oder 5-Nitrosalicylsäure sind;
radioaktives Zirkonium [$^{89}$Zr] wird unter Verwendung der Kohlenstoffmikrokugeln nach der Adsorption kleiner organischer Moleküle adsorbiert, um ein drittes Zwischenprodukt zu erhalten;
die radioaktiven Nuklide zur Behandlung in der gemischten Lösung der Ionen der radioaktiven Nuklide zur Behandlung und der wässrigen Lösung kleiner organischer Moleküle werden unter Verwendung des dritten Zwischenprodukts adsorbiert;
das dritte Zwischenprodukt wird nach der Adsorption der radioaktiven Nuklide zur Behandlung mit einer Natriumphosphatlösung gemischt und umgesetzt, gefolgt von Waschen und Fest-Flüssig-Abscheidung, um ein viertes Zwischenprodukt zu erhalten; und
die erste Lösung wird zum vierten Zwischenprodukt hinzugefügt, und sie werden gleichmäßig gemischt und sterilisiert.

6. Verfahren nach Anspruch 4 oder 5, wobei der erste pH-Wert 1-14, vorzugsweise 3,5-6,5, beträgt; und der zweite pH-Wert 1-14, vorzugsweise 3,5-7,5, beträgt.

7. Sichtbar gemachte radioaktive Kohlenstoffmikrokugelsuspension, die durch das Verfahren nach einem der Ansprüche 4-6 hergestellt wird.

8. Verwendung der sichtbar gemachten radioaktiven Kohlenstoffmikrokugelsuspension nach einem der Ansprüche 1-3 und 7 zum Herstellen eines Mittels zur Behandlung sichtbar gemachter Tumore, wobei der Tumor unter anderem solide Tumore einschließlich Leberkrebs, Bauchspeicheldrüsenkrebs, Nierenkrebs, Brustkrebs, Schilddrüsenkrebs und Darmkrebs sowie orthopädische Tumore umfasst.

**Revendications**

1. Suspension de microsphères de carbone radioactif visualisée, chaque 1 ml de la solution comprenant : 10-500 mg de microsphères de carbone, 5-500 mCi d'activité de nucléides radioactifs pour le traitement, 0,1-100 mCi d'activité de nucléides radioactifs pour l'imagerie, 0-100 mg de petites molécules organiques, et 0,1-1,0 ml d'une première solution, et dans laquelle le nucléide radioactif pour le traitement est choisi parmi l'un quelconque de l'yttrium [$^{90}$Y], du lutétium [$^{177}$Lu], de l'holmium [$^{166}$Ho], du samarium [$^{153}$Sm] et des isotopes de ceux-ci, le nucléide radioactif pour l'imagerie est le zirconium [$^{89}$Zr], et les petites molécules organiques est l'acide 5-sulfosalicylique ou l'acide 5-nitrosalicylique.

2. Suspension selon la revendication 1, dans laquelle les microsphères de carbone sont des matières carbonées sphériques ou non sphériques riches en micropores et mésopores préparées par n'importe quel procédé, avec un diamètre de 0,05-1000 $\mu$m, de préférence 20-60 $\mu$m.

3. Suspension selon la revendication 1, dans laquelle la première solution comprend, mais n'est pas limitée à, une solution d'éthanol, une solution de polyéthylène glycol, et une solution de glycérine ; des solutions d'hydrate de carbone hydrosoluble telles qu'une solution de glucose, une solution de dextrane, et une solution de glucane ; des solutions de cellulose hydrosoluble telles qu'une solution de carboxyméthylcellulose sodique, une solution de carboxyéthylcellulose sodique, et une solution d'hydroxypropyl cellulose ; des solutions d'amidon hydrosoluble telles qu'une solution d'hydroxyéthylamidon et une solution de carboxyméthylamidon sodique ; et d'autres solutions de petite molécule ou polymère hydrosoluble avec des structures similaires.

4. Procédé pour préparer la suspension de microsphères de carbone radioactif visualisée selon l'une quelconque des revendications 1-3, le procédé comprenant les étapes suivantes :

   une solution de nucléides radioactifs pour le traitement est mélangée uniformément avec une solution aqueuse de petites molécules organiques présentant un premier pH, dans lequel le nucléide radioactif pour le traitement est choisi parmi l'un quelconque de l'yttrium [$^{90}$Y], du lutétium [$^{177}$Lu], de l'holmium [$^{166}$Ho], du samarium [$^{153}$Sm] et des isotopes de ceux-ci, et les petites molécules organiques est l'acide 5-sulfosalicylique ou l'acide 5-nitrosalicylique ;
   les nucléides radioactifs pour le traitement dans la solution mélangée ci-dessus est adsorbé au moyen de microsphères de carbone ;
   les microsphères de carbone après l'adsorption des nucléides radioactifs pour le traitement sont mélangées et amenées à réagir avec une solution de phosphate de sodium, suivi par un lavage et une séparation solide-liquide, pour obtenir un premier intermédiaire ;
   le premier intermédiaire est mélangé uniformément avec une solution aqueuse de petites molécules organiques présentant un premier pH pour obtenir une première solution d'intermédiaire et dans lequel les petites molécules organiques est l'acide 5-sulfosalicylique ou l'acide 5-nitrosalicylique ;
   une solution ionique de zirconium radioactif [$^{89}$Zr] présentant un deuxième pH est ajoutée à la première solution d'intermédiaire pour obtenir un deuxième intermédiaire ; et
   la première solution est ajoutée au deuxième intermédiaire, et ils sont mélangés uniformément et stérilisés.

5. Procédé pour préparer la suspension de microsphères de carbone radioactif visualisée selon l'une quelconque des revendications 1-3, le procédé comprenant les étapes suivantes :

   des petites molécules organiques dans une solution aqueuse de petites molécules organiques présentant un premier pH sont adsorbées au moyen de microsphères de carbone et dans lequel les petites molécules organiques est l'acide 5-sulfosalicylique ou l'acide 5-nitrosalicylique ;
   du zirconium radioactif [$^{89}$Zr] est adsorbé au moyen des microsphères de carbone après l'adsorption de petites molécules organiques pour obtenir un troisième intermédiaire ;
   les nucléides radioactifs pour le traitement dans la solution mélangée des ions de nucléides radioactifs pour le traitement et la solution aqueuse de petites molécules organiques sont adsorbés au moyen du troisième intermédiaire ;
   le troisième intermédiaire après l'adsorption des nucléides radioactifs pour le traitement est mélangé et amené à réagir avec une solution de phosphate de sodium, suivi par un lavage et une séparation solide-liquide, pour obtenir un quatrième intermédiaire ; et
   la première solution est ajoutée au quatrième intermédiaire, et ils sont mélangés uniformément et stérilisés.

**6.** Procédé selon la revendication 4 ou 5, dans lequel le premier pH est 1-14, de préférence 3,5-6,5 ; et le deuxième pH est 1-14, de préférence 3,5-7,5.

**7.** Suspension de microsphères de carbone radioactif visualisée préparée par le procédé selon l'une quelconque des revendications 4-6.

**8.** Utilisation de la suspension de microsphères de carbone radioactif visualisée selon l'une quelconque des revendications 1-3 et 7 pour préparer un agent pour le traitement d'une tumeur visualisé, dans laquelle la tumeur comprend, mais n'est pas limitée à des tumeurs solides comportant le cancer du foie, le cancer du pancréas, le cancer du rein, le cancer du sein, le cancer de la thyroïde et le cancer intestinal, et les tumeurs orthopédiques.

FIG. 1

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107715124
- CN 107715122

- WO 2009129577 A1